# EUROPEAN PATENT SPECIFICATION

(11) **EP 1 748 800 B1**
(45) Date of publication and mention of the grant of the patent: **22.10.2008**
(21) Application number: 05743997.8
(22) Date of filing: 11.05.2005
(51) Int. Cl.: A61L 9/01

(54) **ODOUR REDUCTION COMPOSITIONS**
GERUCHSREDUZIERENDE ZUSAMMENSETZUNGEN
COMPOSITIONS DESODORISANTES

(30) Priority: 12.05.2004 GB 0410583; 26.02.2005 GB 0504003
(43) Date of publication of application: 07.02.2007
(73) Proprietor: Quest International Services B.V., 1411 GP Naarden (NL)
(72) Inventor: PERRING, Keith, Douglas, Ashford Kent (GB); RICHARDSON, Anne, Canterbury Kent CT4 7TA (GB); GOULDING, Christine, Margaret, Ashford, Kent TN24 0NX (GB); OSMONT, Jean-Pascal, 27800 Saint Pierre de Salerne (FR)
(74) Representative: Simmons, John Murray
(86) International application number: PCT/IB2005/001616
(87) International publication number: WO 2005/110499

(56) References cited:
- EP-A- 0 890 355
- EP-A- 1 133 982
- WO-A-00/37117
- US-A- 4 113 645
- US-A- 5 089 162
- US-A- 5 698 253
- US-A- 5 888 961

## Description

### FIELD OF INVENTION

According to the invention there is provided a hair-treatment composition comprising an odor-reducing composition for counteracting malodorous amines, wherein the odor-reducing composition comprises at least one odor-reducing material selected from the group consisting of phenyl ethyl methyl ether, Cyprisate, Camonal and paracresyl methyl ether.

### BACKGROUND

There are numerous sources of malodorous amines and a persistent problem is the pungent odor that is produced by them.

Typical sources of malodorous amines include landfills, cat litter, chicken coops, water treatment plants and ponds, garbage, dog kennels, rendering plants, food processing plants, wool plants, fish canneries, sewers, paper mills and rest rooms.

Typical examples of products containing malodorous amines are hair-treatment products such as hair bleaches and colorants.

Hair bleaching and coloring compositions normally contain oxidative agents such as inorganic peroxygen oxidizing agents, and they may additionally contain peroxide activating agents such as aqueous hydrogen peroxide solutions.

Unfortunately, the action of hydrogen peroxide solutions is slow and their use in the treatment of hair may solubilize and decolorize melanin leading to hair damage and undesirable qualities such as brittleness. Also, in order to maintain the necessary level of activity for the aqueous hydrogen peroxide solution, it is necessary that the final hair treatment composition has a pH of from about 9 to about 12. Below a pH of 9, insufficient bleaching action occurs; above pH 12 an excessive amount of hair damage occurs.

To minimize damage to the hair and to increase the reaction rate, an activator is normally used.
Ammonia is known to be an effective activator of the hair-bleaching action of aqueous peroxide solutions. It operates by accelerating the oxidative destruction of particles of hair pigment while its pH adjusting properties maintain the required alkalinity. Therefore, it has become common practice to use ammonia both for its activator effect and for its pH adjusting effect. As a result, the vast majority of hair-bleaching and/or coloring products contain substantial quantities of ammonia.

Unfortunately, the inclusion of ammonia and its derivatives (such as alkanolamines) in hair bleaching and/or coloring products introduces a strong, offensive and difficult to mask malodorous amine odor and provides a hostile environment within which it is difficult to achieve effective perfuming. For example, EP 0890355 discusses the unpleasant odor given off by ammonia when used in hair bleaching or dyeing compositions.

Therefore, there is a need for stable compositions to suppress malodorous amines, particularly the odors associated with ammonia in hair-treatment products.

European Patent Specification No. 1133982 describes perfume materials containing a phenyl ring moiety having an air diffusion coefficient of >5.7 or a C-5 ring moiety, which also contains at least 1 carbon which is sp2 hybridized having an air-diffusion coefficient of >4.4 for use in ammonia-containing products.

WO 00/37117 describes various compositions having a reduced malodour which contain, inter alia 5-methyl-2-(2-methylpropyl)-1,3-dioxane (hereinafter referred to as "Camonal") or methyl 1, 4-dimethylcyclohexylcarboxylate (hereinafter referred to as "Cyprisate") as fragrance materials.

US 5698253 discloses perfume compositions and perfumed products containing Cyprisate as a fragrance material.

US 5888961 discloses Camonal and its use as a fragrance material in perfume compositions and perfumed products.

US 4113645 discloses bleach compositions including perfume compositions for masking chlorine malodours, including perfumes containing paracresyl methyl ether and beta-phenyl ethyl methyl ether.

US 5089162 discloses cleaning compositions that may include paracresyl methyl ether.

### SUMMARY OF THE INVENTION

Preferably, the odor-reducing composition comprises at least two odor-reducing materials selected from the group consisting of phenyl ethyl methyl ether, Cyprisate, Camonal and paracresyl methyl ether.

Suitably, the odor reducing material is present at 10 weight % or above. Advantageously, the odor reducing material is present at 15 weight % or above. More preferably, the odor reducing material is present at 30 weight % or above.

Preferably, the hair-treatment composition is a hair colorant. Alternatively, the hair-treatment composition is a bleaching composition for hair.

In one embodiment of the invention, the malodorous amine comprises ammonia. Suitably, the ammonia is present in an amount from 0.5 weight % to 5 weight % in the hair treatment composition.

According to the invention there is also provided a method of counteracting malodorous amines comprising adding to the source of the malodor a malodor counteracting amount of an odor-reducing composition comprising at least one odor-reducing material selected from the group consisting of phenyl ethyl methyl ether, Cyprisate, Camonal and paracresyl methyl ether.

Preferably, the malodour counteracting amount of the odor-reducing composition comprises at least two odor-reducing materials selected from the group consisting of phenyl ethyl methyl ether, Cyprisate, Camonal and paracresyl methyl ether.

Suitably the odor reducing material is present in said odor-reducing composition at 10 weight % or above. Preferably, the odor reducing material is present in said odor-reducing composition at 15 weight % or above. More preferably, the odor reducing material is present in said odor-reducing composition at 30 weight % or above.

Advantageously, the malodorous amine comprises ammonia in the method of the invention. In one embodiment, the source of the malodour comprises a hair-treatment product. Suitably, the hair-treatment product is a hair colorant.
Alternatively, the hair-treatment product is a bleaching composition for hair.

The present invention relates to a hair treatment composition comprising ammonia and an odor-reducing composition for reducing the perception of malodorous amines, wherein such odor-reducing composition comprises at least one odor-reducing material selected from the group consisting of Phenyl ethyl methyl ether, Cyprisate, Camonal and Paracresyl methyl ether.

Although it is known to include Phenyl ethyl methyl ether, Cyprisate, Camonal and Paracresyl methyl ether in fragrance compositions, it was not known that their inclusion in compositions for use in environments containing malodorous amines could impart odor-counteracting properties. In particular, it has been found that the odor-reducing materials are olfactively stable in environments containing relatively high ammonia concentrations such as hair colorants and bleaches.

Moreover, surprisingly, it has now been found that an odor-reducing composition for counteracting malodorous amines comprising at least two odor-reducing materials selected from the group comprising phenyl ethyl methyl ether, Cyprisate, Camonal and paracresyl methyl ether is particularly efficacious.

### DETAILED DESCRIPTION

With the intention of counteracting the unpleasant smells associated with malodorous amines, such as the smell of ammonia from ammonia-containing hair-treatment products, an investigation was carried out into the effectiveness of a variety of fragrance materials.

The following fragrance materials were tested at the dilutions (v/v) shown. The perceived intensity of each fragrance material was measured, and the perceived intensity of the combination of each fragrance material and malodorous amine was measured in an enclosed environment.
Allyl amyl glycolate (Q) 1%
Allyl heptanoate 1%
Allyl hexanoate 1%
Amyl salicylate 20%
Benzyl salicylate 20%
Camonal 5%
Cis 3 Hexenol (Green Grass) 10%
Citronellol pure 10%
Citronellyl propionate 5%
Cressanther 3%
Cyprisate 2%
Diphenyl oxide 1%
Dispirone 0.5%
Elintaal 15%
Ethyl hepanoate 1%
Ethyl methyl phenyl glycidate 1%
Ethylene brassylate 20%
Florocyclene 5%
Galaxolide solvent free 20%
Gardocyclene 5%
Geranyl propionate 10%
Hexyl salicylate 10%
Iso eugenyl acetate 0.5%
Lilial 10%
Lixetone (Q) 5%
Methyl benzoate 1%
Methyl ionone alpha iso (Q) 5%
Methyl salicylate 1%
Nonalactone gamma 1%
Paracresyl methyl ether 1%
Petiole 1%
Phenoxyethyl isobutyrate beta 5%
Phenyl ethyl methyl ether 3%
Phenyl ethyl phenylacetate 10%
Phenyl ethyl salicylate 5%
Styrallyl propionate 2%
Tetrahydro geraniol 5%
Tetrahydro myrcenol 5%
Timberol 1%
Undecalactone gamma 1%

To determine the performance of each fragrance ingredient against malodorous amines, the following protocols were used.

It is to be noted that evaluations are carried out by 18 to 24 panelists who have been extensively screened for their olfactory acuity and trained in the methods of evaluation.

### Ingredient Intensity: Evaluation Protocol

Intensity was measured by placing a 1ml sample of fragrance material in a 60ml bottle and having it rated by a trained sensory panel. Each fragrance material was assessed 20 times.

### Ingredient Performance Against Malodorous Amine: Evaluation Protocol

Performance was evaluated by placing a 3ml sample of malodor (0.1N ammonium hydroxide) in an uncapped 15ml wide-mouth jar. This jar was then placed into a 500ml wide-mouth jar containing 1ml of test fragrance material in an uncapped 15 ml wide-mouth jar. The 500 ml jar was then capped and the system was left to equilibrate for 30 minutes.

A trained sensory analysis panel using an established scaling technique measured the perceived intensity of ammonia malodor and fragrance material in each sample.

In individual panel sessions, each panelist assessed four fragrance materials. Each fragrance material was assessed against a malodor control consisting of a 500ml wide-mouth jar containing a 15ml wide-mouth jar containing 3ml (0.1N) ammonium hydroxide and a 15ml wide-mouth jar containing 1ml DEP. A hidden control was also included with the samples in order to check the consistency of scaling. To eliminate the build up of ammonia in the jar headspace, panelists were asked to open the jar and wait 3 seconds before beginning the assessment. A 5 minute rest period was given between each fragrance material in order to minimize fatigue and to adhere to recommended safety standards.

A total of 20 assessments were made of each sample. The results of the assessments were analyzed using Analysis of Variance (ANOVA) and multiple comparison tests.

The single fragrance ingredient intensities, and the malodor intensities and fragrance ingredient intensities in the ingredient/malodor mix, are given in Table 1 below.

**Table 1**

| Product | Ingredient/malodor mix | | Ingredient intensity |
|---|---|---|---|
| | Malodor intensity | Ingredient intensity | |
| Phenyl ethyl methyl ether 3% | 29 | 53 | 67 |
| Cyprisate 2% | 34 | 54 | 55 |
| Camonal 5% | 35 | 56 | 66 |
| Paracresyl methyl ether 1% | 37 | 52 | 71 |
| Cis-3-hexenyl salicylate | 56 | 28 | 24 |

Cis-3-hexenyl salicylate was used as a benchmark reference.

Table 1 shows clearly that the fragrance materials Phenyl ethyl methyl ether, Cyprisate, Camonal and Paracresyl methyl ether act as odor-reducing materials, significantly reducing the perceived intensity of the malodor while retaining their ingredient intensity.

To achieve the highly efficacious odor counteracting and fragrance effect of this invention, it is particularly preferred that compositions for reducing the perception of malodorous amines comprise at least two odor-reducing material selected from the group consisting of Phenyl ethyl methyl ether, Cyprisate, Camonal and Paracresyl methyl ether.

More preferably the compositions for reducing the perception of malodorous amines will comprise at least 10 wt. % of odor reducing materials; even more preferably at least 15 wt. % of odor reducing materials; more preferably still at least 30 wt. % of odor reducing materials.

The compositions for reducing the perception of malodorous amines can then be incorporated into malodorous compositions as required.

As used herein the term 'hair' to be treated may be 'living' (i.e. on a living body) or may be 'non-living' (i.e. in a wig, hairpiece or other aggregation of non-living fibers, such as is used in textiles and fabrics). Mammalian, especially human, hair is preferred. However, wool, fur and other melanin containing fibers are also included.

It is understood that the compositions and method of the present invention are not restricted to any particular physical mode or product form, and may be contained for example and not as the limitation to the present invention, in aqueous and non-aqueous products, foams, powders, granules, gels, aerosols, non-aerosols, waxes, microencapsulated vehicles, phase-change microencapsulated vehicles, and the like.

Compositions of the present invention may be used in a number of malodorous amine containing environments or products. For example, and not as a limitation to the present invention, environments such as land fills, cat litter, chicken coops, water treatment plants and ponds, garbage, dog kennels, rendering plants, food processing plants, wool plants, fish canneries, sewers, paper mills and rest rooms, and products for bathroom care, room freshening, air freshening, pet care, adult incontinence, household cleaning, hair treatment, hard surface cleaning, and the like.

### EXAMPLES

Examples of preferred odor-reduction compositions of the present invention are as follows:

### Example 1

| | % |
|---|---|
| Mandarin Italian Pure | 3.2 |
| Citronellyl nitrile (Q) | 0.8 |
| Tridecen-2-nitrile 10% dep (Q) | 0.4 |
| CYPRISATE CI (Q) | 40.0 |
| Cis-3-hexenyl acetate | 0.4 |
| Ligustral (Q) | 0.8 |
| Efetaal (Q) | 1.6 |
| Lilial | 4.0 |
| Iso Jasmone Pure (Q) | 0.4 |
| Phenyl ethyl alcohol | 36.0 |
| Maceal 10% dpg (Q) | 0.4 |
| Patchouli acid washed (Q) | 4.0 |
| Acetylcedrene (Q) | 4.8 |
| Sandela | 1.6 |
| Heliotropin | 1.6 |

### Example 2

| | % |
|---|---|
| Dihydromyrcenol (Q) | 10.0 |
| Herboxane (Q) | 10.0 |
| CAMONAL CI (Q) | 15.0 |
| Allyl heptanoate | 15.0 |
| Allyl amyl glycolate (Q) | 3.0 |
| Mefrosol (Q) | 15.0 |
| MDJ Super (Q) | 3.0 |
| Florocyclene (Q) | 4.0 |
| Hexyl salicylate (Q) | 5.0 |
| Iso-E-Super | 10.0 |
| Bangalol (Q) | 5.0 |
| Silvanone Supra (Q) | 5.0 |

### Example 3

| | % |
|---|---|
| Para-cresyl methyl ether | 6.0 |
| Camonal (Q) | 2.0 |
| Cyprisate (Q) | 3.0 |
| Isobornyl cyclohexanol | 0.5 |
| Florocyclene | 10.0 |
| Gamma decalactone | 6.0 |
| Ethyl vanillin 10% (DPG) | 0.5 |
| Silvanone (Q) | 2.5 |
| Isoamyl acetate | 4.0 |
| Herbanate (Q) | 1.0 |
| Manzanate (Q) | 1.0 |
| Amyl butyrate | 2.0 |
| Trans-2-hexenyl acetate | 1.0 |
| Ortholate (Q) | 8.0 |
| Herboxane (Q) | 8.0 |
| Phenoxyethanol | 8.0 |
| Phenoxyethyl isobutyrate | 10.0 |
| Beta-ionone | 10.0 |
| Karanal (Q) 10%(DPG) | 0.5 |

While the invention has been described with respect to preferred embodiments and examples, those skilled in the art will readily appreciate that various changes and/or modifications can be made to the invention without departing from the spirit or scope of the invention.

## Claims

1. A hair-treatment composition comprising ammonia and an odor-reducing composition for counteracting malodorous amines, wherein the odor-reducing composition comprises at least one odor-reducing material selected from the group consisting of phenyl ethyl methyl ether, Cyprisate, Camonal and paracresyl methyl ether.

2. A hair-treatment composition according to claim 1, wherein the odor-reducing composition comprises at least two odor-reducing materials selected from the group consisting of phenyl ethyl methyl ether, Cyprisate, Camonal and paracresyl methyl ether.

3. A hair-treatment composition as claimed in claim 1 or 2, wherein the odor-reducing material is present at 10 wt. % or above.

4. A hair-treatment composition as claimed in claim 3, wherein the odor-reducing material is present at 15 wt. % or above.

5. A hair-treatment composition as claimed in claim 4, wherein the odor-reducing material is present at 30 wt. % or above.

6. A hair-treatment composition as claimed in any one of the preceding claims, wherein said composition is a hair colorant.

7. A hair-treatment composition as claimed in any one of claims 1 to 5, wherein said composition is a bleaching composition for hair.

8. A hair-treatment composition as claimed in claim 8, wherein the ammonia is present in an amount from 0.5 wt. % to 5 wt. %.

9. A method of counteracting malodorous amines, comprising adding to the source of the malodor a malodor counteracting amount of an odor-reducing composition comprising at least one odor-reducing material selected from the group consisting of phenyl ethyl methyl ether, Cyprisate, Camonal and paracresyl methyl ether.

10. A method as claimed in claim 9, comprising adding to the source of the malodour a malodour counteracting amount of an odor-reducing composition comprising at least two odor-reducing materials selected from the group consisting of phenyl ethyl methyl ether, Cyprisate, Camonal and paracresyl methyl ether.

11. A method as claimed in claim 9 or 10, wherein said odor-reducing material is present in said odor-reducing composition at 10 wt. % or above.

12. A method as claimed in claim 11, wherein said odor-reducing material is present in said odor-reducing composition at 15 wt. % or above.

13. A method as claimed in claim 12, wherein said odor-reducing material is present in said odor-reducing composition at 30 wt. % or above.

14. A method as claimed in any one of claims 9 to 13, wherein the malodorous amine comprises ammonia.

15. A method as claimed in any one of claims 9 to 14, wherein the source of the malodour comprises a hair-treatment product.

16. A method as claimed in claim 15, wherein the hair-treatment product comprises a hair-colorant

17. A method as claimed in claim 15, wherein the hair-treatment product is a bleaching composition for hair.

18. Use of an odour-reducing composition comprising at least one odour-reducing material selected from the group consisting of phenyl ethyl methyl ether, Cyprisate, Camonal and paracresyl methyl ether, for counteracting malodourous amines.

19. The use according to claim 18 wherein the odour-reducing composition comprises at least two odour-reducing materials selected from the group consisting of phenyl ethyl methyl ether, Cyprisate, Camonal and paracresyl methyl ether.

20. The use as claimed in claim 18 or 19, wherein said odor-reducing material is present in said odor-reducing composition at 10 wt. % or above.

21. The use as claimed in claim 20, wherein said odor-reducing material is present in said odor-reducing composition at 15 wt. % or above.

22. The use as claimed in claim 21, wherein said odor-reducing material is present in said odor-reducing composition at 30 wt. % or above.

23. The use as claimed in any one of claims 18 to 22', wherein the malodorous amine comprises ammonia.

24. The use as claimed in any one of claims 18 to 23, wherein the source of the malodour comprises a hair-treatment product.

25. The use as claimed in claim 24, wherein the hair-treatment product comprises a hair-colorant.

26. The use as claimed in claim 24, wherein the hair-treatment product is a bleaching composition for hair.

## Patentansprüche

1. Haarbehandlungszusammensetzung, umfassend Ammoniak und eine Geruch-reduzierende Zusammensetzung, um schlecht riechenden Aminen entgegenzuwirken, wobei die Geruch-reduzierende Zusammensetzung wenigstens ein Geruch-reduzierendes Material umfasst, das ausgewählt ist aus der Gruppe, bestehend aus Phenylethylmethylether, Cyprisat, Camonal und para-Cresylmethylether.

2. Haarbehandlungszusammensetzung nach Anspruch 1, wobei die Geruch-reduzierende Zusammensetzung wenigstens zwei Geruch-reduzierende Materialien umfasst, die ausgewählt sind aus der Gruppe, bestehend aus Phenylethylmethylether, Cyprisat, Camonal und para-Cresylmethylether.

3. Haarbehandlungszusammensetzung, wie sie in Anspruch 1 oder 2 beansprucht wird, worin das Geruch-reduzierende Material mit 10 Gew.-% oder darüber vorliegt.

4. Haarbehandlungszusammensetzung, wie sie in Anspruch 3 beansprucht wird, worin das Geruch-reduzierende Material mit 15 Gew.-% oder darüber vorliegt.

5. Haarbehandlungszusammensetzung, wie sie in Anspruch 4 beansprucht wird, worin das Geruch-reduzierende Material mit 30 Gew.-% oder darüber vorliegt.

6. Haarbehandlungszusammensetzung, wie sie in einem der vorangehenden Ansprüche beansprucht wird, worin die Zusammensetzung ein Haarfärbemittel ist.

7. Haarbehandlungszusammensetzung, wie sie in einem der Ansprüche 1 bis 5 beansprucht wird, worin die Zusammensetzung eine Bleichzusammensetzung für Haar ist.

8. Haarbehandlungszusammensetzung, wie sie in Anspruch 8 beansprucht wird, worin das Ammoniak in einer Menge von 0,5 Gew.-% bis 5 Gew.-% vorliegt.

9. Verfahren, um schlecht riechenden Aminen entgegenzuwirken, umfassend Zu geben zu der Quelle des schlechten Geruchs eine dem schlechten Geruch entgegenwirkende Menge einer Geruch-reduzierenden Zusammensetzung, die wenigstens ein Geruch-reduzierendes Material umfasst, das ausgewählt ist aus der Gruppe, bestehend aus Phenylethylmethylether, Cyprisat, Camonal und para-Cresylmethylether.

10. Verfahren, wie es in Anspruch 9 beansprucht wird, umfassend Zugeben zu der Quelle des schlechten Geruchs eine dem schlechten Geruch entgegenwirkende Menge einer Geruch-reduzierenden Zusammensetzung, die wenigstens zwei Geruch-reduzierende Materialien umfasst, die ausgewählt sind aus der Gruppe, bestehend aus Phenylethylmethylether, Cyprisat, Camonal und para-Cresylmethylether.

11. Verfahren, wie es in Anspruch 9 der 10 beansprucht wird, worin das Geruch-reduzierende Material in der Geruch-reduzierenden Zusammensetzung mit 10 Gew.-% oder darüber vorliegt.

12. Verfahren, wie es in Anspruch 11 beansprucht wird, worin das Geruch-reduzierende Material in der Geruch-reduzierenden Zusammensetzung mit 15 Gew.-% oder darüber vorliegt.

13. Verfahren, wie es in Anspruch 12 beansprucht wird, worin das Geruch-reduzierende Material in der Geruch-reduzierenden Zusammensetzung mit 30 Gew.-% oder darüber vorliegt.

14. Verfahren, wie es in einem der Ansprüche 9 bis 13 beansprucht wird, worin das schlecht riechende Amin Ammoniak umfasst.

15. Verfahren, wie es in einem der Ansprüche 9 bis 14 beansprucht wird, worin die Quelle des schlechten Geruchs ein Haarbehandlungsprodukt umfasst.

16. Verfahren, wie es in Anspruch 15 beansprucht wird, worin das Haarbehandlungsprodukt ein Haarfärbemittel umfasst.

17. Verfahren, wie es in Anspruch 15 beansprucht wird, worin das Haarbehandlungsprodukt eine Bleichzusammensetzung für Haar ist.

18. Verwendung einer Geruch-reduzierenden Zusammensetzung, die wenigstens ein Geruch-reduzierendes Material umfasst, das ausgewählt ist aus der Gruppe, bestehend aus Phenylethylmethylether, Cyprisat, Camonal und para-Cresylmethylether, um schlecht riechenden Aminen entgegenzuwirken.

19. Verwendung nach Anspruch 18, wobei die Geruch-reduzierende Zusammensetzung wenigstens zwei Geruch-reduzierende Materialien umfasst, die ausgewählt sind aus der Gruppe, bestehend aus Phenylethylmethylether, Cyprisat, Camonal und para-Cresylmethylether.

20. Verwendung, wie sie in Anspruch 18 oder 19 beansprucht wird, wobei das Geruch-reduzierende Material in der Geruch-reduzierenden Zusammensetzung mit 10 Gew.-% oder darüber vorliegt.

21. Verwendung, wie sie in Anspruch 20 beansprucht wird, wobei das Geruch-reduzierende Material in der Geruch-reduzierenden Zusammensetzung mit 15 Gew.-% oder darüber vorliegt.

22. Verwendung, wie sie in Anspruch 21 beansprucht wird, wobei das Geruch-reduzierende Material in der Geruch-reduzierenden Zusammensetzung mit 30 Gew.-% oder darüber vorliegt.

23. Verwendung, wie sie in einem der Ansprüche 18 bis 22 beansprucht wird, wobei das schlecht riechende Amin Ammoniak umfasst.

24. Verwendung, wie sie in einem der Ansprüche 18 bis 23 beansprucht wird, wobei die Quelle des schlechten Geruchs ein Haarbehandungsprodukt umfasst.

25. Verwendung, wie sie in Anspruch 24 beansprucht wird, wobei das Haarbehandungsprodukt ein Haarfärbemittel umfasst.

26. Verwendung, wie sie in Anspruch 24 beansprucht wird, wobei das Haarbehandlungsprodukt eine Bleichzusammensetzung für Haar ist.

## Revendications

1. Composition de traitement capillaire comprenant de l'ammoniac et une composition réduisant les odeurs pour neutraliser les amines malodorantes, dans laquelle la composition réduisant les odeurs comprend au moins un matériau réduisant les odeurs choisi dans le groupe constitué par le phényléthylméthyl éther, le Cyprisate, le Camonal et le paracrésylméthyl éther.

2. Composition de traitement capillaire selon la revendication 1, dans laquelle la composition réduisant les odeurs comprend au moins deux matériaux réduisant les odeurs choisis dans le groupe constitué par le phényléthylméthyl éther, le Cyprisate, le Camonal et le paracrésylméthyl éther.

3. Composition de traitement capillaire selon la revendication 1 ou 2, dans laquelle le matériau réduisant les odeurs est présent à 10 % en poids ou plus.

4. Composition de traitement capillaire selon la revendication 3, dans laquelle le matériau réduisant les odeurs est présent à 15 % en poids ou plus.

5. Composition de traitement capillaire selon la revendication 4, dans laquelle le matériau réduisant les odeurs est présent à 30 % en poids ou plus.

6. Composition de traitement capillaire selon l'une quelconque des revendications précédentes, dans laquelle ladite composition est un colorant pour les cheveux.

7. Composition de traitement capillaire selon l'une quelconque des revendications 1 à 5, dans laquelle ladite composition est une composition décolorante pour les cheveux.

8. Composition de traitement capillaire selon la revendication 7, dans laquelle l'ammoniac est présent en une quantité de 0,5 % en poids à 5% en poids.

9. Procédé de neutralisation des amines malodorantes, comprenant l'ajout à la source de mauvaises odeurs, d'une quantité neutralisant les mauvaises odeurs d'une composition réduisant les odeurs comprenant au moins un matériau réduisant les odeurs choisi dans le groupe constitué par le phényléthylméthyl éther, le Cyprisate, le Camonal et le paracrésylméthyl éther.

10. Procédé selon la revendication 9, comprenant l'ajout à la source de mauvaises odeurs, d'une quantité neutralisant les mauvaises odeur d'une composition réduisant les odeurs comprenant au moins deux matériaux réduisant les odeurs choisis dans le groupe constitué par le phényléthylméthyl éther, le Cyprisate, le Camonal et le paracrésylméthyl éther.

11. Procédé selon la revendication 9 ou 10, dans lequel ledit le matériau réduisant les odeurs est présent dans ladite composition réduisant les odeurs à 10 % en poids ou plus.

12. Procédé selon la revendication 1 l, dans lequel ledit matériau réduisant les odeurs est présent dans ladite composition réduisant les odeurs à 15 % en poids ou plus.

13. Procédé selon la revendication 12, dans lequel ledit matériau réduisant les odeurs est présent dans ladite composition réduisant les odeurs à 30 % en poids ou plus.

14. Procédé selon l'une quelconque des revendications 9 à 13, dans lequel l'amine malodorante comprend l'ammoniac.

15. Procédé selon l'une quelconque des revendications 9 à 14, dans lequel la source de mauvaises odeurs comprend un produit de traitement capillaire.

16. Procédé selon la revendication 15, dans lequel le produit de traitement capillaire comprend un colorant pour les cheveux.

17. Procédé selon la revendication 15, dans lequel le produit de traitement capillaire est une composition décolorante pour les cheveux.

18. Utilisation d'une composition réduisant les odeurs comprenant au moins un matériau réduisant les odeurs choisi dans le groupe constitué par le phényléthylméthyl éther, le Cyprisate, le Camonal et le paracrésylméthyl éther, pour neutraliser les amines malodorantes.

19. Utilisation selon la revendication 18, dans laquelle la composition réduisant les odeurs comprend au moins deux matériaux réduisant les odeurs choisis dans le groupe constitué par le phényléthylméthyl éther, le Cyprisate, le Camonal et le paracrésylméthyl éther.

20. Utilisation selon la revendication 18 ou 19, -dans laquelle ledit le matériau réduisant les odeurs est présent dans ladite composition réduisant les odeurs à 10 % en poids ou plus.

21. Utilisation selon la revendication 20, dans laquelle ledit matériau réduisant les odeurs est présent dans ladite composition réduisant les odeurs à 15 % en poids ou plus.

22. Utilisation selon la revendication 21, dans laquelle ledit matériau réduisant les odeurs est présent dans ladite composition réduisant les odeurs à 30 % en poids ou plus.

23. Utilisation selon l'une quelconque des revendications 18 à 22, dans laquelle l'amine malodorante comprend l'ammoniac.

24. Utilisation selon l'une quelconque des revendications 18 à 23, dans laquelle la source de mauvaises odeurs comprend un produit de traitement capillaire.

25. Utilisation selon la revendication 24, dans laquelle le produit de traitement capillaire comprend un colorant pour les cheveux.

26. Utilisation selon la revendication 24, dans laquelle le produit de traitement capillaire est une composition décolorante pour les cheveux.
